# EUROPEAN PATENT SPECIFICATION

(11) **EP 2 376 168 B1**
(45) Date of publication and mention of the grant of the patent: **06.11.2013**
(21) Application number: 09796987.7
(22) Date of filing: 01.12.2009
(51) Int. Cl.: A61M 16/06, A62B 17/04

(54) **HOOD FOR NON-INVASIVE VENTILATION OF PATIENTS**
HAUBE ZUR NICHTINVASIVEN BEATMUNG VON PATIENTEN
CLOCHE DE HOOD POUR VENTILATION NON INVASIVE DE PATIENTS

(30) Priority: 03.12.2008 IT MI20080396 U
(43) Date of publication of application: 19.10.2011
(60) Divisional application: 13185978.7
(73) Proprietor: Intersurgical S.P.A., 41037 Mirandola, Modena (IT)
(72) Inventor: NAVALESI, Paolo, I-28100 Novara (IT); OLIVIERI, Carlo, I-28100 Novara (IT); ROSSI, Paolo, I-41037 Mirandola (IT)
(74) Representative: Bonfreschi, Mario
(86) International application number: PCT/EP2009/066172
(87) International publication number: WO 2010/063733

(56) References cited:
- EP-A1- 1 852 137
- EP-A2- 1 279 411
- WO-A2-03/097145
- WO-A2-2007/128571
- US-A- 4 620 538
- US-A- 5 819 728
- US-B1- 6 854 459

## Description

### Technical Field

The present invention relates to a hood for non-invasive ventilation of patients.

### Background Art

As is known, non-invasive ventilation of patients in general already uses hoods which, generally speaking, are provided by a substantially cylindrical container body with or without an upper cushion; the inlet and outlet connectors for the air and oxygen are connected to said body, and the hood is provided with a collar constituted by a thin membrane made of elastically flexible plastic material, which mates with the neck of the patient in order to provide the seal.

Such collar is typically provided by an extremely flexible film that adheres to the skin of the patient without applying pressure.

Such flexibility and elasticity which, on the one hand, is particularly useful for providing the seal, on the other hand, constitutes a technical limitation of the hood, since it can reduce the effectiveness of the ventilation due to the fact that the collar is deformed elastically and raises the hood, requiring the physician to set therapies with higher pressure values.

Moreover, it can lead to a lack of synchronization during the beginning of the spontaneous respiratory act of the patient and the response of the ventilator, which is essential for correct ventilation therapy.

This aspect therefore influences directly the functionality of the hood, with the need to provide armpit restraining systems, such as for example ordinary padded straps designed to keep the hood in position.

The alternating swelling of the soft collar inevitably causes the upward movement of the hood, and therefore a traction is applied which constitutes a pressure of the straps under the patient's armpits, which in the long term inevitably inconveniences the patient.

Conventional ventilation treatment hoods are disclosed in US 5 819 728 A and US 6 854 459 B1.

### Disclosure of the Invention

The aim of the invention is to solve the problem described above, by providing a hood for non-invasive ventilation of patients that allows to reduce drastically the effect of the elasticity or compliance caused by the flexible collar, further achieving a reduction in the movement of the hood during the respiratory act.

Within this aim, an object of the invention is to provide an increase in the stiffness of the structure of the hood, so that it can be far less influenced by the sudden pressure variations that occur within the respiration circuit.

Another object of the present invention is to obtain a correct synchronization between the beginning of the spontaneous respiratory act of the patient and the response of the ventilator, thus obtaining a correct ventilation therapy.

Another object of the present invention is to provide a hood which, thanks to its particular constructive characteristics, is capable of giving the greatest assurances of reliability and safety in use.

In accordance with the invention, there is provided a hood for non-invasive ventilation of patients, as defined in the appended claims.

### Brief Description of the Drawings

Further characteristics and advantages of the present invention will become better apparent from the description of a preferred but not exclusive embodiment of a hood for non-invasive ventilation of patients, illustrated by way of non-limiting example in the accompanying drawings, wherein:
Figure 1 is a schematic exploded and partially sectional view of the hood, with an annular body provided by two hinged elements;
Figure 2 is a schematic view of the hood with an inner inflatable cushion which is applied or heat-sealed to the annular body;
Figure 3 is a view of the hood in the position for use;
Figure 4 is a bottom plan view of the hood;

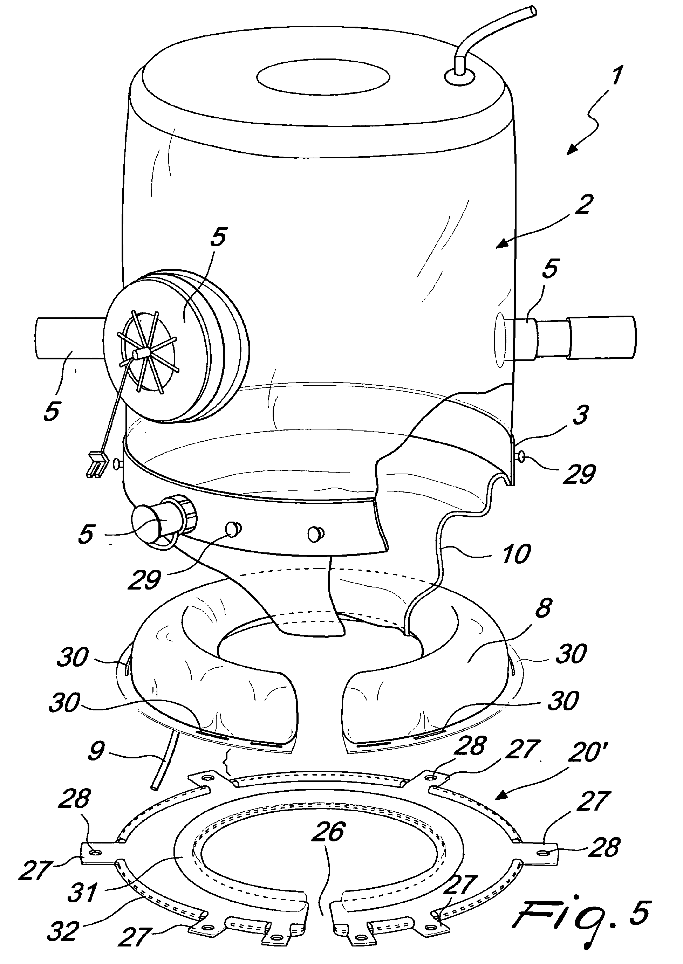

Figure 5 is an exploded view of the hood with an annular body provided in one piece;

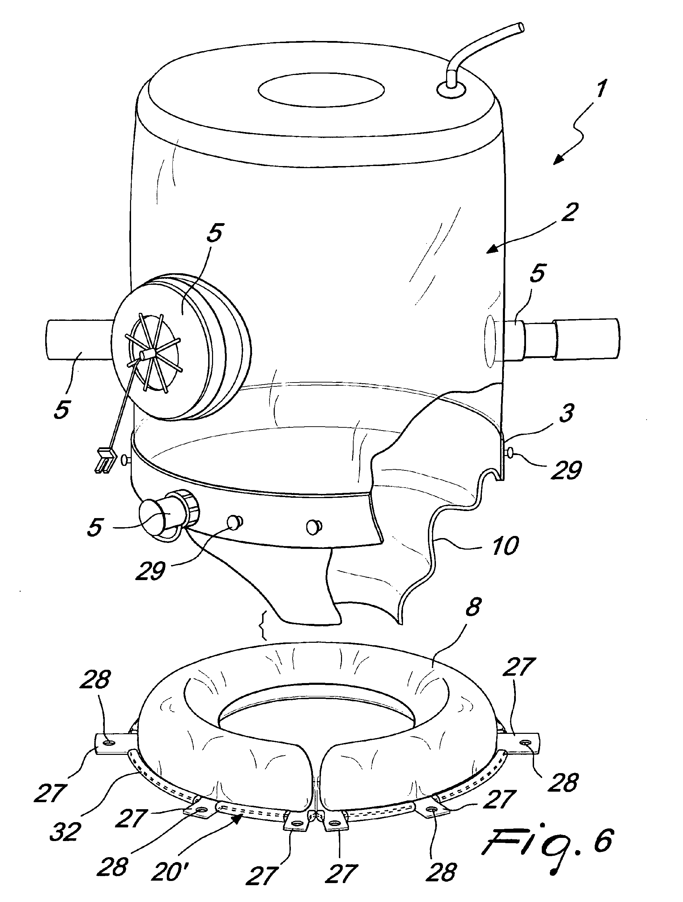

Figure 6 is a view of the lower cushion applied or heat-sealed to the monolithic annular body; and

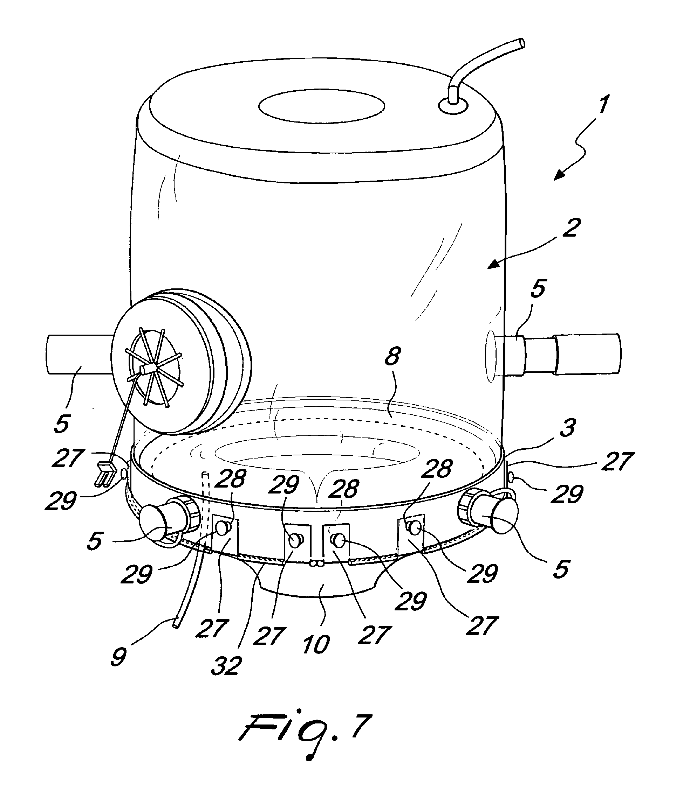

Figure 7 is a view of the hood with the monolithic annular body shown assembled.

### Ways of carrying out the Invention

With reference to the figures, the hood for non-invasive ventilation of patients in general, generally designated by the reference numeral 1, comprises a containment body 2, which is advantageously provided by a cylindrical element made of optically transparent material, which though being flexible cannot expand. The containment body 2, at its lower open end, is connected to a rigid base ring 3, to which a collar 10 is connected which advantageously is made of flexible material in order to be mated hermetically to the neck of the patient, whose head is accommodated in the containment body 2.

As is common practice, the containment body is provided with intake and discharge connectors and with optional openings which can be closed for access to the interior and so forth, all generically designated by the reference numeral 5.

Moreover an inner cushion is provided, generally designated by the reference numeral 8, which can be inflated with an external connector 9, which is positioned below the collar 10, fixed or heat-sealed on the annular body 20, moreover , the cushion 8 can be made of an inflatable material or can be made of a soft material fixed or heat-sealed to the annular body.

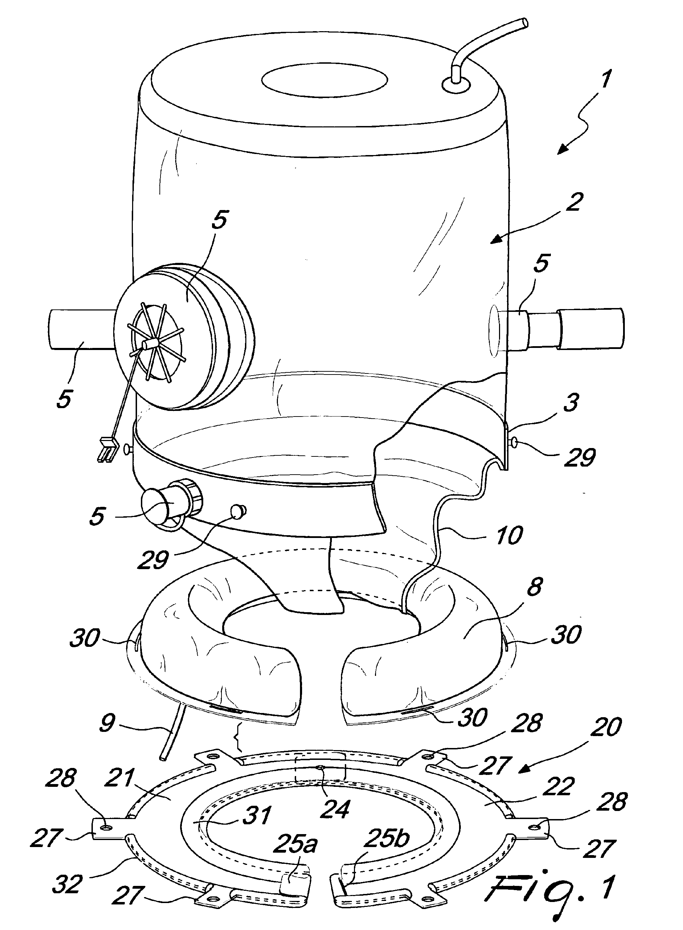

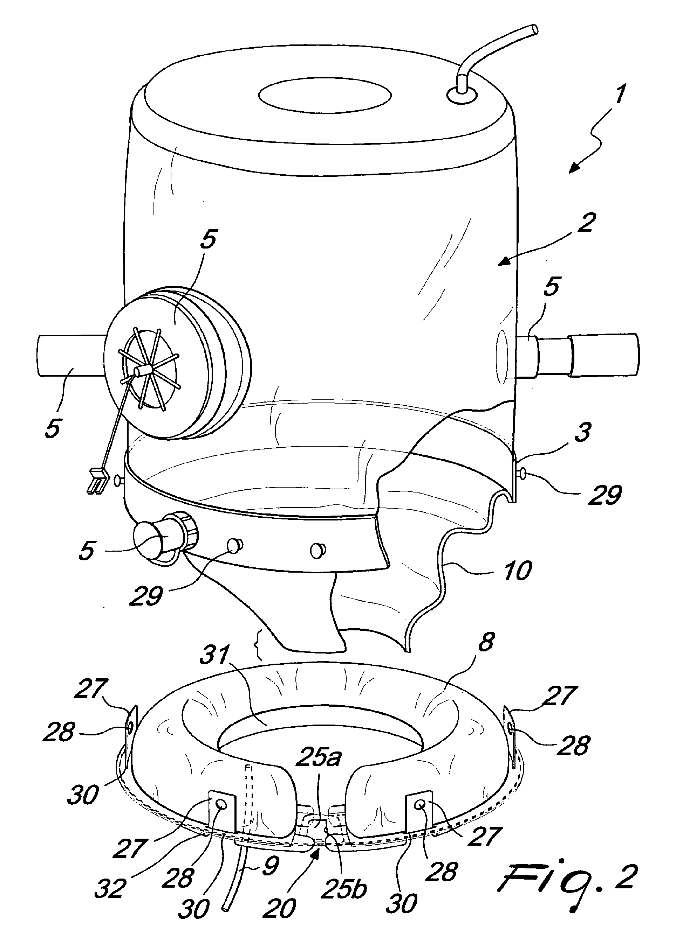

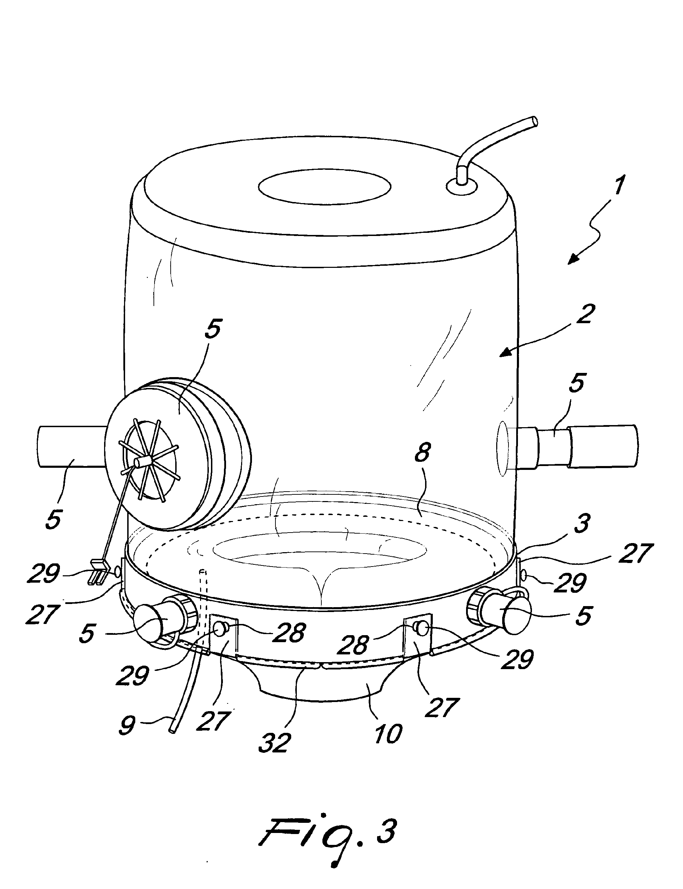

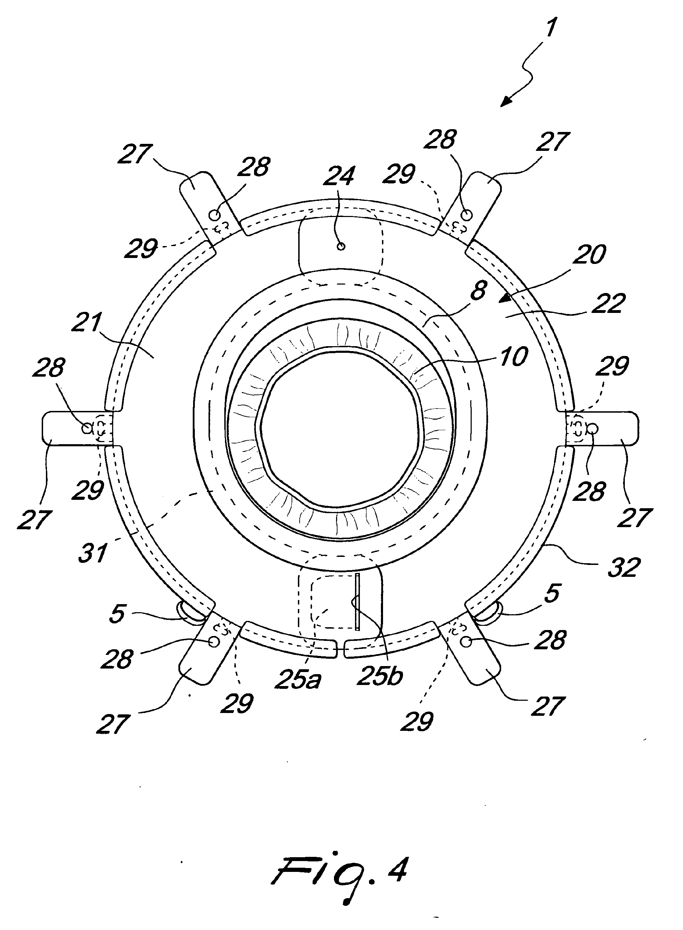

The peculiarity of the invention consists in that an annular body, designated by the reference numeral 20, is provided which, in the embodiment shown in Figures 1 to 4, is constituted by two semicircles 21 and 22, which are connected one another by pivoting means constituted by a pivoting pin 24 and have, at the other end, joining means constituted by a tab 25a arranged on one end which can be inserted in a slot 25b arranged on the facing end.

The annular body can be provided by means of substantially rigid elements or optionally by means of elements that have a certain flexibility and are advantageously provided by means of a sheet made of plastic material such as polyvinyl chloride, polypropylene, acrylonitrile-butadienestyrene, polyoxymethylene, known commercially by the trade name Delrin, or other polymers.

The annular body 20 is provided with means for fixing to the containment body 2, which in a possible embodiment are constituted by radial tabs 27, which are fixed to or heat-sealed or are part of the annular body 20 and have a through hole 28 to provide a stable connection with coupling pins 29 provided at the base of the containment body at the rigid ring 3.

With the flexible element that provides the annular body 20 mated to the containment body, the annular body is substantially rigid and is adapted to act as an abutment element for the collar 10 together with the interposed cushion 8.

According to an embodiment that is linked conceptually to the preceding one, the annular body, designated by the reference numeral 20', can be provided in one piece with a notch 26 which can be closed, with the possibility to arrange the annular body around the neck of the patient by utilizing the flexibility of the material.

The tabs 27 can enter coupling slots 30 provided on the cushion 8, or optionally the cushion 8 can be directly heat-sealed or mated on the annular body 20 or 20'.

Moreover, on the inner edge of the annual body 20 or 20', either in two parts or in one piece, there is a soft protective edge 31; optionally, on the outer edge there is an outer protective edge 32.

With the described arrangement, when the annular body is connected stably to the containment body, the collar 10 abuts against the cushion 8 and the abutment plane constituted by the annular body 20, thus obtaining, in a way, a stiffening of the structure of the hood, which prevents the elastic deformation of the collar and consequently prevents the creation both of the upward displacement of the hood and the variation of the inner volume of the containment body, both of which are due to the elastic deformation of the collar.

In practice the collar is retained between the inner cushion 8 and the abutment plane 20, thus providing an optimum seal on the neck of the patient without however causing its elastic deformation, with consequent negative influence on the pressure values during the respiratory act.

From what has been described above it is evident that the invention achieves the proposed aim and objects, and in particular the fact is stressed that the presence of an annular body constituted by an element that is rigid or optionally becomes rigid thanks to its connection to the containment body provides an abutment element for the flexible collar, thus allowing to obtain optimum sealing values without however having the corresponding deformation of the collar, which accordingly does not cause the rise of the hood or the variation of the inner volume of the containment body, with the consequent possibility to avoid using the armpit straps to retain the hood.

The invention thus conceived is susceptible of numerous modifications and variations, all of which are within the scope of the appended claims.

All the details may further be replaced with other technically equivalent elements.

In practice, the materials used, so long as they are compatible with the specific use, as well as the contingent shapes and dimensions, may be any according to requirements and to the state of the art.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A hood (1) for non-invasive ventilation of patients, comprising a containment body (2) provided with at least one optically transparent portion and with a collar (10) that can be mated hermetically to the neck of the patient whose head is accommodated within said containment body (2), the hood further comprising an annular body (20, 20') which can be associated around the neck and can be fixed to said containment body (2), said annular body (20, 20') when fixed to said containment body being substantially rigid, the hood being **characterized in that** said collar (10) is connected to a rigid base ring (3) connected to the lower open end of said containment body (2) and said collar (10) extends from said base ring (3) downwardly through and below said annular body (20, 20') which acts as an abutment element for said collar (10).

2. The hood (1) according to claim 1, **characterized in that** said annular body (20) is constituted by two semicircles (21, 22), which are connected to one another at one end and are provided at the other end with joining means.

3. The hood (1) according to claim 2, **characterized in that** said semicircles (21, 22) are substantially rigid.

4. The hood (1) according to claim 2, **characterized in that** said two semicircles (21, 22) are made of substantially flexible material, adapted to be stiffened by the connection to said containment body (2).

5. The hood (1) according to one or more of the preceding claims 2-4, **characterized in that** said joining means are constituted by a tab (25a), which is arranged at one end of one of said semicircles (21, 22) and can be inserted in a slot (25b) arranged on a facing end of the other semicircle (21, 22).

6. The hood (1) according to claim 4, **characterized in that** said substantially flexible semicircles (21, 22) are made of a sheet of plastic material.

7. The hood (1) according to one or more of the preceding claims, **characterized in that** it comprises fixing means (27, 28 and 29) for fixing said annular body (20, 20') to said containment body (2).

8. The hood (1) according to claim 7, **characterized in that** said fixing means are constituted by radial tabs (27) that protrude from said annular body (20, 20') and are provided with a through hole (28) for connection to a corresponding coupling pin (29) which is provided correspondingly at said rigid ring (3) arranged at the base of said containment body (2).

9. The hood (1) according to claim 8, **characterized in that** it comprises at least one inner cushion (8), which can engage at least one face of said collar (10), said at least one cushion (8) being provided with coupling slots (30) for the passage of said radial tabs (27).

10. The hood (1) according to one or more of the preceding claims 1-8, **characterized in that** it comprises at least one inner cushion (8), which can engage at least one face of said collar (10) and is jointly associated with said annular body (20, 20').

11. The hood (1) according to one or more of the preceding claims 1 and 7-9, **characterized in that** said annular body (20') is constituted by a single piece provided with a notch (26).

12. The hood (1) according to one or more of the preceding claims, **characterized in that** it comprises, on the inner edge of said annular body (20, 20'), a soft protective edge (31).

13. The hood (1) according to one or more of the preceding claims, **characterized in that** it comprises, on the outer edge of said annular body (20, 20'), an outer protective edge (32).

## Patentansprüche

1. Haube (1) zur nicht invasiven Beatmung von Patienten, einen Umgebungskörper (2) umfassend, der mit mindestens einem optisch durchlässigen Abschnitt und einer Manschette (10) ausgestattet ist, die hermetisch an den Hals des Patienten angelegt werden kann, dessen Kopf im Umgebungskörper (2) untergebracht ist, wobei die Haube ferner einen ringförmigen Körper (20, 20') umfasst, der um den Hals herum angebracht und am Umgebungskörper (2) befestigt werden kann, wobei der ringförmige Körper (20, 20') im Wesentlichen starr ist, wenn er am Umgebungskörper befestigt ist, wobei die Haube **dadurch gekennzeichnet ist, dass** die Manschette (10) an einen starren Basisring (3) angeschlossen ist, der am unteren offenen Ende des Umgebungskörpers (2) angeschlossen ist, und dass sich die Manschette (10) vom Basisring (3) abwärts durch und bis unter den ringförmigen Körper (20, 20') erstreckt, der als ein Anschlagelement für die Manschette (10) dient.

2. Haube (1) nach Anspruch 1, **dadurch gekennzeichnet, dass** der ringförmige Körper (20) aus zwei Halbkreisen (21, 22) gebildet ist, die an einem Ende aneinander angeschlossen sind und am anderen Ende mit Verbindungsmitteln ausgestattet sind.

3. Haube (1) nach Anspruch 2, **dadurch gekennzeichnet, dass** die Halbkreise (21, 22) im Wesentlichen starr sind.

4. Haube (1) nach Anspruch 2, **dadurch gekennzeichnet, dass** die zwei Halbkreise (21, 22) aus im Wesentlichen flexiblem Material hergestellt sind, das dafür eingerichtet ist, durch den Anschluss an den Umgebungskörper (2) versteift zu werden.

5. Haube (1) nach einem der Ansprüche 2 bis 4, **dadurch gekennzeichnet, dass** die Verbindungsmittel aus einer Lasche (25a) gebildet sind, die an einem Ende eines der Halbkreise (21, 22) angeordnet ist und in einen Schlitz (25b) eingeführt werden kann, der am gegenüberliegenden Ende des anderen Halbkreises (21, 22) angeordnet ist.

6. Haube (1) nach Anspruch 4, **dadurch gekennzeichnet, dass** die im Wesentlichen flexiblen Halbkreise (21, 22) aus einer Bahn aus Kunststoffmaterial hergestellt sind.

7. Haube (1) nach einem der vorhergehenden Ansprüche, **dadurch gekennzeichnet, dass** sie Befestigungsmittel (27, 28 und 29) zum Befestigen des ringförmigen Körpers (20, 20') am Umgebungskörper (2) umfasst.

8. Haube (1) nach Anspruch 7, **dadurch gekennzeichnet, dass** die Befestigungsmittel aus radial angeordneten Laschen (27) gebildet sind, die aus dem ringförmigen Körper (20, 20') hervorstehen und mit einer durchgehenden Öffnung (28) für den Anschluss an einen entsprechenden Kupplungsstift (29) ausgestattet sind, der übereinstimmend am starren Ring (3) bereitgestellt ist, der an der Basis des Umgebungskörpers (2) angeordnet ist.

9. Haube (1) nach Anspruch 8, **dadurch gekennzeichnet, dass** sie mindestens ein inneres Polster (8) umfasst, das sich an mindestens eine Fläche der Manschette (10) anlegen kann, wobei das mindestens eine Polster (8) mit Kupplungsschlitzen (30) für den Durchtritt der radial angeordneten Laschen (27) ausgestattet ist.

10. Haube (1) nach einem oder mehreren der vorhergehenden Ansprüche 1 bis 8, **dadurch gekennzeichnet, dass** sie mindestens ein inneres Polster (8) umfasst, das sich an mindestens eine Fläche der Manschette (10) anlegen kann und verbindend am ringförmigen Körper (20, 20') angebracht ist.

11. Haube (1) nach einem oder mehreren der vorhergehenden Ansprüche 1 und 7 bis 9, **dadurch gekennzeichnet, dass** der ringförmige Körper (20') aus einem einzelnen Stück gebildet ist, das mit einer Aussparung (26) ausgestattet ist.

12. Haube (1) nach einem oder mehreren der vorhergehenden Ansprüche, **dadurch gekennzeichnet, dass** sie am Innenrand des ringförmigen Körpers (20, 20') einen weichen Schutzrand (31) umfasst.

13. Haube (1) nach einem der vorhergehenden Ansprüche, **dadurch gekennzeichnet, dass** sie am Außenrand des ringförmigen Körpers (20, 20') einen äußeren Schutzrand (32) umfasst.

## Revendications

1. Cloche de Hood (1) pour la ventilation non invasive de patients, comprenant un corps de contention (2) pourvu d'au moins une partie optiquement transparente et d'une collerette (10) qui peut être ajustée hermétiquement au cou du patient, dont la tête est placée dans ledit corps de contention (2), la cloche de Hood comprenant en outre un corps annulaire (20, 20') qui peut être associé autour du cou et fixé audit corps de contention (2), ledit corps annulaire (20, 20') étant essentiellement rigide lorsqu'il est fixé audit corps de contention, la cloche de Hood étant **caractérisée en ce que** ladite collerette (10) est reliée à un anneau de base rigide (3) connecté à l'extrémité ouverte inférieure dudit corps de contention (2), et **en ce que** ladite collerette (10) s'étend à partir dudit anneau de base (3) vers le bas à travers ledit corps annulaire (20, 20') et en dessous de celui-ci, qui fait office d'élément de butée pour ladite collerette (10).

2. Cloche de Hood (1) selon la revendication 1, **caractérisée en ce que** ledit corps annulaire (20) est constitué de deux demi-cercles (21, 22) qui sont connectés l'un à l'autre à une extrémité et sont pourvus à l'autre extrémité d'un moyen de jonction.

3. Cloche de Hood (1) selon la revendication 2, **caractérisée en ce que** lesdits demi-cercles (21, 22) sont essentiellement rigides.

4. Cloche de Hood (1) selon la revendication 2, **caractérisée en ce que** lesdits deux demi-cercles (21, 22) sont constitués d'un matériau essentiellement flexible adapté pour être rigidifié par la connexion audit corps de contention (2).

5. Cloche de Hood (1) selon une ou plusieurs des revendications précédentes 2 à 4, **caractérisée en ce que** ledit moyen de jonction est constitué par une languette (25a) qui est agencée à une extrémité d'un desdits demi-cercles (21, 22) et peut être insérée dans une rainure (25b) agencée dans une extrémité qui lui fait face de l'autre demi-cercle (21, 22).

6. Cloche de Hood (1) selon la revendication 4, **caractérisée en ce que** lesdits demi-cercles essentiellement flexibles (21, 22) sont constitués d'une feuille de matériau plastique.

7. Cloche de Hood (1) selon une ou plusieurs des revendications précédentes, **caractérisée en ce qu'**elle comprend un moyen de fixation (27, 28 et 29) pour fixer ledit corps annulaire (20, 20') audit corps de contention (2).

8. Cloche de Hood (1) selon la revendication 7, **caractérisée en ce que** ledit moyen de fixation est constitué de languettes radiales (27) qui font saillie dudit corps annulaire (20, 20') et sont pourvues d'un trou traversant (28) pour assurer la connexion à une broche d'accouplement correspondante (29) qui est pourvue de manière correspondante sur ledit anneau rigide (3) agencé à la base dudit corps de contention (2).

9. Cloche de Hood (1) selon la revendication 8, **caractérisée en ce qu'**elle comprend au moins un coussin interne (8) qui peut s'engager avec au moins une face de ladite collerette (10), ledit au moins un coussin (8) étant pourvu de rainures d'accouplement (30) pour le passage desdites languettes radiales (27).

10. Cloche de Hood (1) selon une ou plusieurs des revendications précédentes 1 à 8, **caractérisée en ce qu'**elle comprend au moins un coussin interne (8) qui peut s'engager avec au moins une face de ladite collerette (10) et est associé conjointement audit corps annulaire (20, 20').

11. Cloche de Hood (1) selon une ou plusieurs des revendications précédentes 1 et 7 à 9, **caractérisée en ce que** ledit corps annulaire (20') est constitué d'une pièce unique pourvue d'une entaille (26).

12. Cloche de Hood (1) selon une ou plusieurs des revendications précédentes, **caractérisée en ce qu'**elle comprend un bord protecteur mou (31) sur le bord interne dudit corps annulaire (20, 20').

13. Cloche de Hood (1) selon une ou plusieurs des revendications précédentes, **caractérisée en ce qu'**elle comprend un bord protecteur externe (32) sur le bord externe dudit corps annulaire (20, 20').
